(19) Europäisches Patentamt
European Patent Office
Office européen des brevets

(11) Publication number: **0 185 225 B1**

(12) **EUROPEAN PATENT SPECIFICATION**

(45) Date of publication of patent specification: **03.01.90**

(51) Int. Cl.[5]: **C 07 F 15/00,** A 61 K 31/28

(21) Application number: **85114932.8**

(22) Date of filing: **26.11.85**

(54) **Platinum complexes of aliphatic tricarboxylic acid.**

(30) Priority: **17.12.84 US 682951**

(43) Date of publication of application:
**25.06.86 Bulletin 86/26**

(45) Publication of the grant of the patent:
**03.01.90 Bulletin 90/01**

(84) Designated Contracting States:
**AT BE CH DE FR GB IT LI NL SE**

(56) References cited:
**EP-A-0 039 272**

**CHEMICO-BIOLOGICAL INERACTIONS, vol. 46, no. 2, 1983, pages 219-232, Elsevier Scientific Publishers Ireland Ltd., Ireland; B. DAS SARMA et al.: "Platinum complexes with anticancer potential and their evaluation by a colorimetric lambda prophage induction assay"**

(73) Proprietor: **AMERICAN CYANAMID COMPANY**
**1937 West Main Street P.O. Box 60**
**Stamford Connecticut 06904-0060 (US)**

(72) Inventor: **Bitha, Panayota**
**128 Country Club Lane**
**Pomona New York 10970 (US)**
Inventor: **Child, Ralph Grassing**
**432 Erhardt Road**
**Pearl River New York 10965 (US)**
Inventor: **Hlavka, Joseph John**
**Pine Hill Road**
**Tuxedo New York 10987 (US)**
Inventor: **Lin, Yang-I**
**4 Pelham Court**
**Nanuet New York 10954 (US)**

(74) Representative: **Wächtershäuser, Günter, Dr.**
**Tal 29**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 185 225 B1

## Description

This invention is concerned with new compounds of the formula:

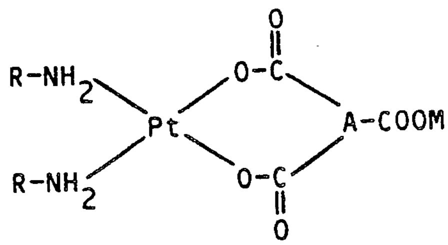

wherein M is cationic hydrogen, sodium or potassium and R is hydrogen or alkyl ($C_1$—$C_5$) or when taken together are selected from the group consisting of moieties of the formulae:

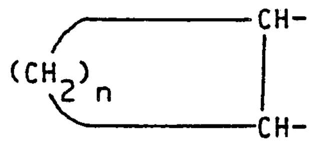
,
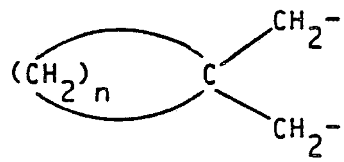
,
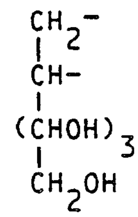

and

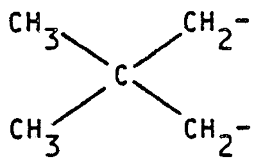

wherein n is the integer 3—5, inclusive, and A is selected from the group consisting of moieties of the formulae:

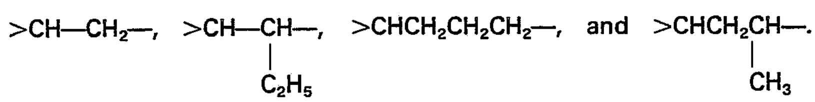

The compounds of this invention may be prepared according to the following flowchart.

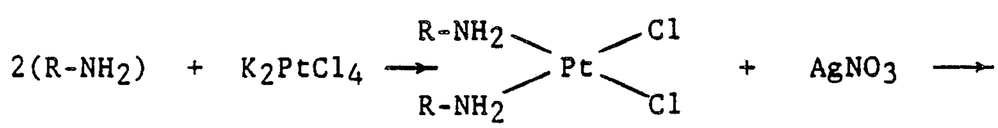

(1) (2) (3)

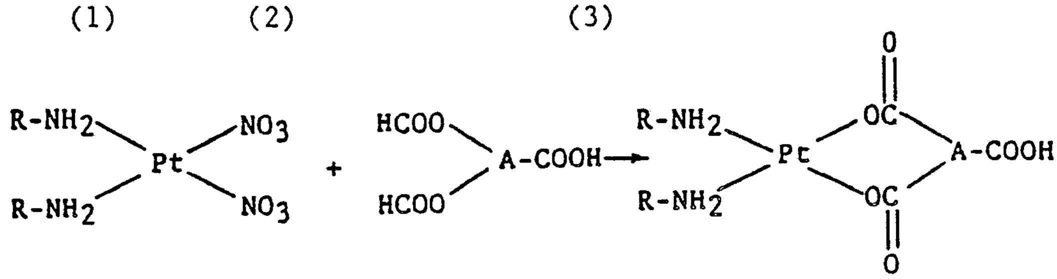

According to the above flowchart, and amine (1) where R is as described above is reacted with potassium tetrachloroplatinate (2) in water giving platinum complex (3) which is then reacted with silver nitrate giving a solution of the platinum nitrate complex (4). The complex (4) is then reacted with a solution of a tricarboxylic acid derivative (5) in the presence of two equivalents of sodium hydroxide, where A is as described above, in an aqueous base giving the product (6).

In EP—A—39 272, platinum complexes of trisodium isocitrate are described. This type of ligand is bound to Pt with only one of its carboxylate groups and with its OH-group in 1-position. Such structure is

principally different from the structure of the presently claimed compounds.

Lymphocytic Leukemia P388 Test

The animals used were BDF/1 mice, all of one sex, weighing a minimum of 18 g and all within a 3 g weight range. There were 5 or 6 animals per test group. The tumor transplant was by intraperitoneal injection of 0.5 of dilute ascitic fluid containing $10^6$ cells of lymphocytic leukemia P388. The test compounds were administered intraperitoneally on days 1, 5 and 9 relative to tumor inoculation, at various doses. The animals were weighed and the survivors recorded on a regular basis for 30 days. The median survival time and the ratio of survival time for treated (T)/control (C) animals were calculated. The positive control compound was Cisplatin. The results of this test with representative compounds of this invention appear in Table I.

TABLE I
Lymphocytic Leukemia P388 Test

| Compound | Dose (mg/kg) | Median Survival (Days) | T/C × 100 (%) |
|---|---|---|---|
| 2,2-dimethyl-1,3-propanediamine, compound with [1,1,2-ethanetricarboxylato(2-)-$O^1O^1$]platinum (1:1) | 100 | 20.5 | 178 |
| | 50 | 20.5 | 178 |
| | 25 | 19.0 | 165 |
| | 12.5 | 16.0 | 139 |
| | 6.2 | 14.5 | 126 |
| Control | — | 11.5 | — |
| Cisplatin | 1 | 29.0 | 252 |
| | 0.25 | 29.0 | 165 |
| | 0.06 | 15.5 | 135 |
| *trans*-(−)-1,2-cyclohexanediamine compound with[1,1,2-ethanetricarboxylato(2-)-$O^1O^1$]platinum (1:1) | 100 | 22.5 | 214 |
| | 50 | 19.0 | 181 |
| | 25 | 14.0 | 133 |
| | 12 | 15.0 | 143 |
| | 6 | 11.0 | 105 |
| Control | — | 10.5 | — |
| Cisplatin | 1 | 22.0 | 210 |
| | 0.25 | 15.0 | 143 |
| | 0.26 | 13.0 | 124 |
| 1,1-cyclobutanedimethanamine, compound with [1,1,2-ethanetricarboxylato (2-)-$O^1O^1$]-platinum (1:1) | 100 | 19.5 | 193 |
| | 50 | 17.5 | 173 |
| | 25 | 15.5 | 153 |
| | 12 | 14.5 | 144 |
| | 6 | 12.5 | 124 |
| | 3 | 12.5 | 124 |
| Control | — | 10.1 | — |
| Cisplatin | 1 | 23 | 228 |
| | 0.25 | 19 | 188 |
| | 0.06 | 16.5 | 163 |
| | 0.015 | 13.5 | 134 |

TABLE I (continued)

| Compound | Dose (mg/kg) | Median Survival (Days) | T/C × 100 (%) |
|---|---|---|---|
| *cis* (and *trans*)-1,2-cyclohexanediamine, compound with [1,1,2-ethanetricarboxylato-(2-)-O¹O¹]platinum (1:1) | 50<br>25<br>12<br>6<br>3<br>1.5 | 18<br>14<br>14.5<br>12<br>15<br>12.5 | 178<br>139<br>144<br>119<br>149<br>124 |
| Control | — | 10.1 | — |
| Cisplatin | 1<br>0.25<br>0.06<br>0.015 | 23<br>19<br>16.5<br>13.5 | 228<br>188<br>163<br>134 |
| 1,2-diamino-1,2-dideoxy-D-glucitol, compound with [1,1,2-ethanetricarboxylato-(2-)-O¹O¹]platinum (1:1) | 100<br>50<br>25<br>12<br>6 | 17.2<br>16.5<br>16.5<br>15<br>15 | 124<br>119<br>119<br>109<br>109 |
| Control | — | 13.8 | — |
| Cisplatin | 1<br>0.25<br>0.06<br>0.015 | 23.5<br>22.0<br>20<br>20 | 170<br>159<br>145<br>145 |
| *trans*(1,2-cyclohexanediamine--*N*-*N*')[1,1,2-ethanetricar-boxylato(3-)O¹O¹]platinate (1-), sodium salt | 50<br>25<br>12.5<br>6.2<br>3.1 | 25<br>22.5<br>20<br>18.5<br>14.5 | 227<br>205<br>182<br>168<br>132 |
| Control | — | 11 | — |
| Cisplatin | 2<br>1 | 15.5<br>26.5 | 141<br>241 |
| (2,2-dimethyl-1,3-propane-diamine-*N,N'*-[1,1,2-ethane-tricarboxylato(3-)-O¹O¹]-platinate(1-), sodium salt | 100<br>50<br>25<br>12.5<br>6.2<br>3.1 | 17<br>20<br>16<br>16<br>12.5<br>13 | 170<br>200<br>160<br>160<br>125<br>130 |
| Control | — | 10 | — |
| Cisplatin | 2<br>1 | 10.5<br>23.5 | 105<br>235 |

Melanotic Melanoma B16

The animals used were C57BC/6 mice, all of the same sex, weighing a minimum of 17 g and all within a 3 g weight range. There were 10 animals per test group. A 1 g portion of melanotic melanoma $B_{16}$ tumor was homogenized in 10 ml of cold balanced salt solution and a 0.5 ml aliquot of the homogenate was implanted intra peritoneally into each of the test mice. The test compounds were administered intraperitoneally on days 1 through 9, relative to tumor inoculation at various doses. The animals were weighed and survivors recorded on a regular basis for 60 days. The median survival time for treated (T)/

control (C) animals were calculated. The positive control compound was Cisplatin. The results of this test on representative compounds of this invention appear in Table II.

TABLE II
Melanotic Melanoma $B_{16}$ Test

| Compound | Dose (mg/kg) | Median Survival (Days) | T/C × 100 (%) |
|---|---|---|---|
| 2,2-dimethyl-1,3-propanediamine, compound with [1,1,2-ethanetricarboxylato(2-)-$O^1O^1$]platinum (1:1) | 100<br>50<br>25<br>12.5<br>6.25 | 25<br>22.5<br>20.5<br>19.5<br>17.5 | 170<br>153<br>139<br>133<br>119 |
| Control | — | 14.7 | — |
| Cisplatin | 0.4<br>0.2<br>0.1<br>0.05 | 18.5<br>21<br>18<br>19 | 126<br>143<br>122<br>129 |
| *trans*-(−)-1,2-cyclohexanediamine, compound with [1,1,2-ethanetricarboxylato(2-)-$O^1O^1$]platinum (1:1) | 25<br>12<br>6<br>3<br>1.5 | 28<br>26.5<br>23.5<br>22.5<br>20.1 | 175<br>166<br>147<br>141<br>126 |
| Control | — | 16 | — |
| Cisplatin | 0.4<br>0.2<br>0.1<br>0.05 | 25<br>25<br>23<br>21.5 | 156<br>156<br>144<br>134 |

Colon 26 Adenocarcinoma Test

The animals used were Balb/C mice all of one sex, weighing a minimum of 17 g and all within a 3 g weight range. There were 5 or 6 mice per test group with three groups of 5 or 6 animals used as untreated controls for each test. The tumor implat was by intraperitoneal or subcutaneous injection of 0.5 ml of a 2% Colon 26 tumor brei in Eagle's MEM medium containing antibiotics. The test compounds were administered intraperitoneally on days 1, 5 and 9 (relative to tumor implant at various doses). The mice were weighed and deaths recorded on a regular basis for 30 days. The median survival times and the ratio of survival time for treated (T)/control (C) animals were calculated. The positive control compound was Cisplatin. The results of this test on representative compounds of this invention appear in Table III.

TABLE III

Colon 26 Adenocarcinoma Test

| Compound | Dose (mg/kg) | Median Survival (Days) | T/C × 100 (%) |
|---|---|---|---|
| *trans*-(−)-1,2-cyclohexanediamine, compound with [1,1,2-ethanetricarboxylato(2-)-$O^1O^1$]platinum (1:1) | 50<br>25 | 18.5<br>22 | 112<br>133 |
| Control | — | 16.5 | — |
| Cisplatin | 1<br>0.5<br>0.25 | 18.5<br>30.5<br>29.5 | 112<br>179<br>179 |
| *cis*(and *trans*)-1,2-cyclohexanediamine, compound with [[2,2′,2″-nitrilotris[acetato]](2-)-O,$O^1$]platinum (1:1) | 100<br>50<br>25 | 30.5<br>20.5<br>22.5 | 185<br>124<br>136 |
| Control | — | 16.5 | — |
| Cisplatin | 1<br>0.5<br>0.25 | 18.5<br>30.5<br>29.5 | 112<br>185<br>179 |

Lymphocytic Leukemia L1210 Test

The animals used were $BDF_1$ or $CD_2F_1$ mice, all of one sex, weighing a minimum 17 g and all within a 3 g weight range. There were 6 mice in each test group and 18 mice in control groups. The tumor transplant was by intraperitoneal injection of 0.5 ml of lymphocytic leukemia L1210 at a concentration of $10^5$ cells per mouse. The test compounds were administered on days 1, 5 and 9 relative to tumor inoculation at various doses. The mice were weighed and survivors recorded on a regular basis for 30 days. The median survival time and the ratio of survival time for treated (T)/control (C) mice were calculated. The positive control compounds were Cisplatin and 5-Fluorouracil given intraperitoneally at the indicated doses. The results of this test on representative compounds of this invention appear in Table IV.

TABLE IV

Lymphocytic Leukemia L1210 Test

| Compound | Dose (mg/kg) | Median Survival (Days) | T/C × 100 (%) |
|---|---|---|---|
| 2,2-dimethyl-1,3-propanediamine, compound with [1,1,2-ethanetricarboxylato (2-)-$O^1$,$O^1$]platinum (1:1) | 100<br>50<br>25<br>12.5 | 12.4<br>27.2<br>27.0<br>12.2 | 151<br>332<br>329<br>149 |
| Control | — | 8.2 | — |
| Cisplatin | 6<br>3<br>1.5 | 12.2<br>16.6<br>11.8 | 149<br>202<br>144 |
| 5-Fluoruracil | 60 | 20.2 | 246 |

TABLE IV (continued)

| Compound | Dose (mg/kg) | Median Survival (Days) | T/C × 100 (%) |
|---|---|---|---|
| *trans*-(−)-1,2-cyclohexane-diamine, compound with [1,1,2-ethane tricarboxylato (2-)-$O^1$,$O^1$]platinum (1:1) | 100<br>50<br>25<br>12.5 | 13.6<br>21.0<br>25.8<br>15.2 | 132<br>204<br>250<br>148 |
| Control | — | 10.3 | — |
| Cisplatin | 6<br>3<br>1.5 | 20.8<br>14.2<br>13.4 | 202<br>138<br>130 |
| 5-Fluorouracil | 60 | 14.8 | 144 |
| 1,1-cyclobutanedimethanamine, compound with [1,1,2-ethane-tricarboxylato (2-)-$O^1$,$O^1$]-platinum (1:1) | 50<br>25<br>12.5 | 23.2<br>22.4<br>13.0 | 252<br>243<br>141 |
| Control | — | 9.2 | — |
| Cisplatin | 6<br>3<br>1.5 | 16.0<br>12.0<br>11.0 | 174<br>130<br>120 |
| 5-Fluorouracil | 60 | 15.4 | 167 |
| *cis*(and *trans*)-1,2-cyclohexanediamine, compound with [1,1,2-ethanetricarboxylato-(2-)-$O^1$,$O^1$]platinum (1:1) | 50<br>25<br>12.5 | 15.0<br>21.8<br>14.8 | 179<br>260<br>176 |
| Control | — | 8.4 | — |
| Cisplatin | 6<br>3<br>1.5 | 16.0<br>12.2<br>10.0 | 190<br>145<br>119 |
| 5-Fluorouracil | 60 | 14.4 | 171 |

Cisplatin Resistant Lymphocytic Leukemia L1210/Cis DPP

The L1210/Cis DPP tumor is a subline of L1210 leukemia, resistant to Cisplatin and maintained as an ascites tumor in DBA/2 mice. The assay for antitumor activity was performed as described above for L1210. The results on representative compounds of this invention appear in Table V.

TABLE V
Ciplatin Resistant Lymphocytic Leukamia
L1210/Cis DPP Test

| Compound | Dose (mg/kg) | Median Survival (Days) | T/C × 100 (%) |
|---|---|---|---|
| 2,2-dimethyl-1,3-propanediamine, compound with [1,1,2-ethanetricarboxylato (2-)-$O^1$,$O^1$]platinum (1:1) | 100<br>50<br>25<br>12.5 | 15.2<br>26.8<br>8.8<br>8.0 | 197<br>348<br>114<br>104 |
| Control | — | 7.7 | — |
| Cisplatin | 6<br>3<br>1.5 | 8.8<br>8.0<br>8.6 | 114<br>104<br>112 |
| 5-Fluorouracil | 60 | 30 | 390 |
| *trans*-(−)-1,2-cyclohexanediamine, compound with [1,1,2-ethanetricarboxylato (2-)-$O^1$,$O^1$]platinum (1:1) | 100<br>50<br>25<br>12.5 | 21.8<br>22.6<br>22.8<br>17.8 | 256<br>266<br>268<br>209 |
| Control | — | 8.5 | — |
| Cisplatin | 6<br>3<br>1.5 | 9.2<br>9.2<br>9.4 | 108<br>108<br>111 |
| 5-Fluorouracil | 60 | 27.2 | 320 |
| 1,1-cyclobutanedimethanamine, compound with [1,1,2-ethanetricarboxylato (2-)-$O^1$,$O^1$]-platinum (1:1) | 100<br>50<br>25<br>12.5 | 11.2<br>17.0<br>10.2<br>10.0 | 127<br>193<br>116<br>114 |
| Control | — | 8.8 | — |
| Cisplatin | 6<br>3<br>1.5 | 8.0<br>9.6<br>8.6 | 91<br>109<br>98 |
| *cis*(and *trans*)-1,2-cyclohexanediamine, compound with [1,1,2-ethanetricarboxylato-(2-)-$O^1$,$O^1$]platinum (1:1) | 50<br>25<br>12.5 | 17.8<br>26.0<br>21.4 | 214<br>313<br>258 |
| Control | — | 8.3 | — |
| Cisplatin | 6<br>3<br>1.5 | 8.2<br>8.4<br>8.2 | 99<br>101<br>99 |
| 5-Fluorouracil | 60 | 19.2 | 231 |

M5076 Sarcoma

The M5076 reticular cell Sarcoma is propagated as subcutaneous (sc) implants in C57B2/6 female mice. In the assays for antitumor activity, $BDF_1$ mice of either sex were inoculated sc with 0.5 ml of a 10% tumor brei. Test compounds were administered ip on days 1, 5, 9, 13 and 17 relative to tumor inoculation on day

zero. Tumor measurements in mm were made by means of a vernier caliper on day 22 relative to tumor implantation and tumor weights in mg estimated by the formula:

$$\frac{\text{length} \times (\text{width})^2}{2}$$

with appropriate T/C values being calculated. The results of this test on representative compounds of this invention appear in Table VI, compared to the results obtained with Cisplatin and Cytoxan.

TABLE VI

M5076 Sarcoma

| Compound | Dose (mg/kg) | Av. Tumor Wt. (mg) | T/C × 100 (%) |
|---|---|---|---|
| 2,2-dimethyl-1,3-propanediamine, compound with [1,1,2-ethanetricarboxylato (2-)-$O^1$,$O^1$]platinum (1:1) | 50 | 0 | 0 |
| | 25 | 0 | 0 |
| | 12.5 | 47 | 2 |
| | 6.2 | 734 | 38 |
| Control | — | 1927 | — |
| Cisplatin | 6.0 | 0 | 0 |
| | 3.0 | 361 | 19 |
| | 1.5 | 865 | 45 |
| *trans*-(−)-1,2-cyclohexanediamine, compound with [1,1,2-ethanetricarboxylato (2-)-$O^1$,$O^1$]platinum (1:1) | 50 | 0 | 0 |
| | 25 | 0 | 0 |
| | 12.5 | 0 | 0 |
| | 6.2 | 103 | 5 |
| Control | — | 1927 | — |
| Cisplatin | 6.0 | 0 | 0 |
| | 3.0 | 361 | 91 |
| | 1.5 | 865 | 45 |

Human Breast (MX—1) Tumor Xenograft

The human breast (MX—1) carcinoma is propagated as subcutaneous (sc) implants in athymic (Balb/c nude) mice. In assays for antitumor activity, athymic (Balb/c nude male mice were implanted sc with four to five $2mm^2$ tumor fragments on day zero. Test compounds were administered intraperitoneally (ip) once every fourth day for a total of three injections starting when tumors were approximately 100 mg in size (staging day, usually 14 days after tumor implantation). Tumor measurements were made in mm by means of a Vernier caliper on day 12 and 16 relative to staging day and tumor weights in mg estimated from the formula

$$\frac{\text{Length} \times (\text{width})^2}{2}.$$

The difference ( ) in mean tumor weight (mean final tumor weight minus mean initial tumor weight) was determined for each test group and the treated (T)/control (C) value expressed in percent. The results of this test on a representative compound of this invention appears in Table VII. The positive control compound was Cisplatin.

TABLE VII

Human Breast (MX—1) Tumor Xenograft

| Compound | Dose (mg/kg) | Days Post Staging 12: ΔTumor Wt (mg) | 12: T/C × 100 (%) | 12: Survivors / Treated | 16: ΔTumor Wt (mg) | 16: T/C × 100 (%) | 16: Survivors / Treated |
|---|---|---|---|---|---|---|---|
| 2,2-dimethyl-1,3-propanediamine, compound with [1,1,2-ethanetricarboxylato-(2-)-$O^1$,$O^1$]platinum (1:1) | 50 | −71 | −56 | 3/4 | | | 0/4 |
| | 25 | −05 | −06 | 3/4 | — | | 0/4 |
| | 12.5 | 68 | 12 | 3/4 | 100 | 12 | 3/4 |
| Control | — | 549 | — | 8/8 | 814 | — | 8/8 |
| Cisplatin | 80 | −31 | −54 | 5/5 | −43 | −75 | 5/5 |

This aspect of the invention includes novel compositions of matter and the method of inducing the regression and/or palliation of leukemia and related cancers in mammals using the novel compounds of this invention when administered in amounts ranging from about 1 mg to about 1.2 g per square meter of body surface area per day. The interrelationship of dosages for animals of various sizes and species and humans (based on mg/$m^2$ of surface area) is described by Freireich, E. J., *et al.*, Quantitative Comparison of Toxicity of Anticancer Agents in Mouse, Rat, Hamster, Dog, Monkey and Man. Cancer Chemother. Rep., *50*, No. 4, 219—244, May 1966. A preferred dosage regimen for optimum results would be from about 3 mg/$m^2$/day to about 200 mg/$m^2$/day, and such dosage units are employed that a total of from about 5 mg to about 360 mg of the active compound for a subject of about 70 kg of body weight are administered in a 24 hour period. This dosage regimen may be adjusted to provide the optimum therapeutic response. For example, several divided doses may be administered daily or the dose may be proportionally reduced as indicated by the exigencies of the therapeutic intravenous, intramuscular or subcutaneous routes.

The active compounds may be administered parenterally. Solutions or dispersions of the active compound can be prepared in water, suitably mixed with a surfactant such as hydroxypropylcellulose. Dispersions can also be prepared in glycerol, liquid polyethylene glycols, and mixtures thereof in oils. Under ordinary conditions of storage and use these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol, liquid polyethylene glycol), suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be obtained by the use in the compositions of agents which delay absorption, for example, aluminum monostearate and gelatin. Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subject to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active cmpound in amounts ranging from about 2 mg to about 2 g, with from about 5 to about 360 mg being preferred. Expressed in proportions, the active compound is generally present in from about 2 to about 100 mg/ml of carrier. In the case of compositions containing supplementary active ingredients, the dosages are determined by reference to the usual dose and manner of administration of the said ingredients.

Regression and palliation of cancers are attained, for example, using intraperitoneal administration. A single intravenous dosage or repeated daily dosages can be administered. Daily dosages up to about 5 or 10 days are often sufficient. It is also possible to dispense one daily dosage or one dose on alternate or less frequent days. As can be seen from the dosage regimens, the amount of principal active ingredient administered is a sufficient amount to aid regression and palliation of the leukemia or the like, in the absence of excessive deleterious side effects of a cytotoxic nature to the host harboring the cancer. As used herein, cancer disease means blood malignancies such as leukemia, as well as other solid and non-solid malignancies such as the melanocarcinomas, lung carcinomas and mammary tumors. By regression and palliation is meant arresting or retarding the growth of the tumor or other manifestation of the disease compared to the course of the disease in the absence of treatment.

This invention will be described in greater detail in conjunction with the following non-limiting specific examples.

Example 1

2,2-Dimethyl-1,3-propanediamine, compound with [1,1,2-ethanetricarboxylato(2-)-$O^1$,$O^1$]platinum (1:1)

To a solution of 12.45 g of potassium tetrachloroplatinate in 60 ml of water was added 3.06 g of 2,2-dimethyl-1,3-propanediamine. This mixture was allowed to stand overnight and then the solid was collected, giving 7.0 g of 2,2-dimethyl-1,3-propanediamine compound with platinum chloride (1:1).

A reaction mixture comprising 49.2 g of triethyl 1,1,2-ethanetricarboxylate, 135 ml of 5N sodium hydroxide and 65 ml of water was stirred at 100°C for 3 hours, then cooled and concentrated to about 150 ml. A 50 ml portion of cold concentrated hydrochloric acid was added and the mixture was extracted three times with ether. The ether extracts were combined, dried and evaporated, giving 12.5 g of 1,1,2-ethanetricarboxylic acid, mp 169—170°C.

To a suspension of 3.68 g of 2,2,2-dimethyl-1,3-propanediamine compound with platinum chloride (1:1 in 30 ml of water was added a solution of 3.40 g silver nitrate in 30 ml of water. This mixture was stirred in the dark for 3 hours and then filtered. To the filtrate was added a solution of 1.62 g of 1,1,2-ethanetricarboxylic acid in 20 ml of 1N sodium hydroxide. This mixture was allowed to stand for 3 hours and then filtered. The filtrate was concentrated almost to dryness and then refrigerated. The resulting solid was collected, washed with water and dried, giving 2.3 g of the desired product as a colorless solid.

Example 2

*trans*-(−)-1,2-Cyclohexanediamine, compound with [1,1,2-ethanetricarboxylato(2-)-$O^1$,$O^1$]platinum (1:1)

A 4.56 g portion of *trans*-1,2-diaminocyclohexane was dissolved in 20 ml of water. To this solution was added a solution of 16.6 g of potassium tetrachloroplatinate in 100 ml of water. After standing 2.5 hours the solid was collected, washed with water and dried, giving 12.98 g of *trans*-(−)-1,2-cyclohexanediamine, compound with platinum chloride (1:1).

A 2 g portion of *trans*-(−)-1,2-cyclohexanediamine, compound with platinum chloride (1:1) was suspended in 20 ml of water and a solution of 1.8 g of silver nitrate in 20 ml of water was added. The suspension was stirred for 3 hours and then filtered. The filtrate was added to a solution of 859 mg of 1,1,2-ethanetricarboxylic acid in 5.3 ml of 2M potassium hydroxide, stirred for 2.5 hours and then evaporated to dryness. The residue was slurried in 5 ml of water.

The solid was collected with cold water and dried, giving 868 mg of the desired product.

Example 3

1,1-Cyclobutanedimethanamine, compound with [1,1,2-ethanetricarboxylato(2-)-$O^1$,$O^1$]platinum (1:1)

A mixture of 20 g of 1,3-dibromopropane, 6.6 g of malononitrile and 27.6 g of potassium carbonate in 400 ml of acetonitrile was refluxed on a steam bath for 20 hours and then filtered while hot. The filtrate was concentrated to dryness, giving an oil which was taken up in methylene chloride, extracted three times with water, dried and concentrated to a dark yellow oil. This oil was vacuum distilled, giving at 41—44°C, 0.4 mm, 6.0 g of 1,1-cyclobutanedicarbonitrile as semi-solid.

A mixture of 10.6 g of 1,1-cyclobutanedicarbonitrile and 150 ml of tetrahydrofuran was treated dropwise and rapidly with 300 ml of 1N borane in tetrahydrofuran. The reaction was cooled in an ice bath, then stirred at room temperature overnight. A 125 ml portion of ethanol was added dropwise, the reaction was stirred for 12 hours, filtered and filtrate concentrated to dryness. The residue was dissolved in 100 ml of water, basified with 6N sodium hydroxide and extracted with methylene chloride. The extract was dried, evaporated, taken up in ether and treated with 33 ml of 6N hydrochloric acid in ispropanol. The resulting solid was recrystallized from 40 ml of methanol containing 3 drops of 6N hydrochloric acid in isopropanol and 20 ml of isopropanol, giving 2.74 g of 1,1-cyclobutanedimethanamine dihydrochloride mp 240—245°C.

A filtered solution of 1.87 g of 1,1-cyclobutanedimethanamine dihydrochloride in 30 ml of water was treated with 1.64 g of sodium acetate followed by 4.15 g of potassium tetrachloroplatinate. This mixture was filtered after one hour and the filtrate allowed to stand 24 hours and refiltered. This filtrate, after standing 24 hours, gave 430 mg of 1,1-cyclobutanedimethanamine, compound with platinum chloride (1:1) mp 280°C dec.

A 1.35 g portion of 1,1-cyclobutanedimethanamine compound with platinum chloride was suspended in 10 ml of water and a solution of 1.02 g of silver nitrate in 10 ml of water was added. This mixture was

stirred overnight, then filtered and the filtrate stirred with a solution of 486 mg of 1,1,2-ethanetricarboxylic acid in 6 ml of 1N sodium hydroxide. This mixture was allowed to stand 48 hours, the solid was collected, washed with cold water and dried, giving 800 mg of the desired product as colorless crystals, mp 228—230°C (dec.).

Example 4

*cis*(and *trans*)-1,2-Cyclohexanediamine, compound with [1,1,2-ethanetricarboxylato(2-)-O[1],O[1]]platinum (1:1)

To a solution of 2.43 g of 1,1,2-ethanetricarboxylic acid in 20 ml of water and 3 ml of 10N sodium hydroxide was added a solution of 5.09 g silver nitrate in 10 ml of water. The resulting suspension was stirred in the dark for 8 hours and the solid collected, washed with water and dried, giving 4.3 g of 1,1,2-ethanetricarboxylic acid, disilver salt.

A suspension of 1.14 g of 1,2-cyclohexanediamine compound with platinum chloride (1:1), 1.21 g of 1,1,2-ethanetricarboxylic acid, disilver salt and 100 ml of water was stirred overnight and then filtered. The filtrate was evaporated to dryness giving 1.19 g of the desired product.

Example 5

Diamine, compound with [1,1,2-ethanetricarboxylato(2-)-O[1],O[1]]platinum (1:1)

A 1.0 g portion of Cisplatin was suspended in 5 ml of water and treated with a solution of 1.14 g of silver nitrate in 5 ml of water. This mixture was stirred for 2 hours, then filtered through diatomaceous earth. The filtrate was treated with a solution of 535 mg of 1,1,2-ethanetricarboxylic acid in 6.7 ml of 1N sodium hydroxide and stirred for 2 hours. The solid 7stirred for 2 hours. The solid was collected, washed with water and dried, giving 300 mg of the desired product, mp 229—230°C (dec.).

Example 6

1,2-Diamino-1,2-dideoxy-D-glucitol, compound with [1,1,2-ethanetricarboxylato(2-)O[1],O[1]]platinum (1:1)

A solution of 20 g of D-glucosamine hydrochloride in 20 ml of glacial acetic acid and 80 ml of water was heated on a steam bath for 1 hour. After cooling, the formed osazone was filtered off and discarded. The clear filtrate was reduced under 50 lb pressure in a Parr hydrogenator containing 10 ml of Raney nickel catalyst. After the reduction was complete, the catalyst was filtered off and the filtrate was complete, the catalyst was filtered off and the filtrate stirred with carbon, refiltered and extracted three times with toluene. The remaining aqueous solution was then treated with 0.68 g of sodium bicarbonate followed by a suspension of 3.32 g of potassium tetrachloroplatinate in 16 ml of water and then stirred overnight. The resulting yellow-brown solid was filtered off and recrystallized from hot water giving yellow crystals mp 262—264°C, of 1,2-diamino-1,2-dideoxy-D-glucitol, compound with platinum dichloride (1:1).

A suspension of 1.34 g of 1,2-diamino-1,2-dideoxy-D-glucitol, compound with platinum chloride (1:1) in 10 ml of water was treated with a solution of 1.02 g of silver nitrate in 10 ml of water. After 1 hour the mixture was filtered and the filtrate treated with a solution of 1,1,2-ethanetricarboxylic acid in 6 ml of 1N sodium hydroxide. After standing 3 days the solution was filtered and the filtrate treated with one volume of isopropanol. The resulting solid was collected and dried, giving 0.64 g of the desired product mp 195—200°C.

Example 7

*Trans*-(1,2-Cyclohexanediamine-*N,N'*)[1,1,2-ethanetricarboxylato(3-)O[1],O[1]]platinate(1-), sodium salt

A 3.52 g portion of *trans*-(−)-1,2-cyclohexanediamine, compound with [1,1,2-ethanetricarboxylato(2-)-O[1],O[1]]platinum (1:1) was suspended in 30 ml of water and a solution of 630 mg of sodium bicarbonate in 25 ml of water was added with stirring. The cloudy solution was filtered and the filter washed with 5 ml of water. The combined filtrate and wash was freeze-dried, giving 3.7 g of the desired product.

Example 8

2,2-Dimethyl-1,3-propanediamine-*N,N'*)[1,1,2-ethanetricarboxylato(3-)O[1],O[1]]platinate(1-), sodium salt

A 2.058 g portion of 2,2-dimethyl-1,3-propanediamine, compound with [1,1,2-ethanetricarboxylato-(2-)O[1],O[1]] platinum (1:1) was suspended in 5 ml of water and a solution of 378 mg of sodium bicarbonate in 10 ml of water was added. The resulting solution was filtered, degassed under a vacuum pump and freeze-dried, giving 1.92 g of the desired product.

**Claims for the Contracting States: BE CH DE FR GB IT LI NL SE**

1. A compound of the formula:

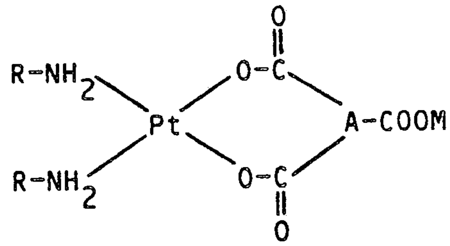

wherein M is hydrogen, sodium or potassium and R is hydrogen or alkyl($C_1$—$C_5$) or when taken together are selected from the group consisting of those of the formulae:

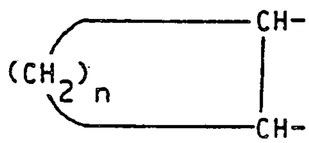,
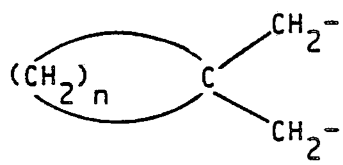,
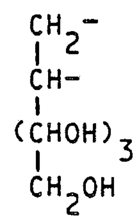

and
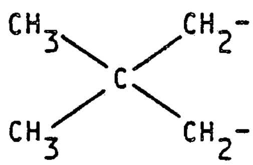

wherein n is the integer 3—5, inclusive; and A is selected from the group consisting of those of the formulae:

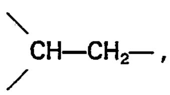,
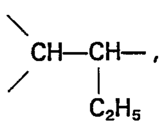,
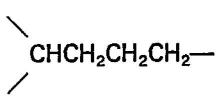
and
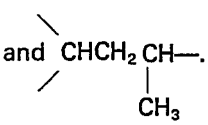.

2. The compound according to Claim 1, 2,2-dimethyl-1,3-propanediamine, compound with [1,1,2-ethanetricarboxylato(2-)-O[1],O[1]]platinum (1:1).

3. The compound according to Claim 1, *trans*-(1)-1,2-cyclohexanediamine, compound with [1,1,2-ethanetricarboxylato(2-)-O[1],O[1]]platinum (1:1).

4. The compound according to Claim 1, 1,1-cyclobutanedimethanamine, compound with [1,1,2-ethanetricarboxylato(2-)-O[1],O[1]]platinum (1:1).

5. The compound according to Claim 1, *cis*(and *trans*)-1,2-cyclohexanediamine, compound with [1,1,2-ethanetricarboxylato(2-)-O[1],O[1]]platinum (1:1).

6. A process of producing a compound of the formula:

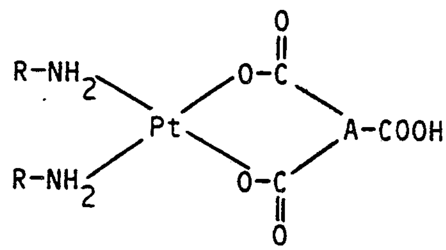

wherein R is hydrogen or alkyl($C_1$—$C_5$) or when taken together are selected from the group consisting of those of the formalae:

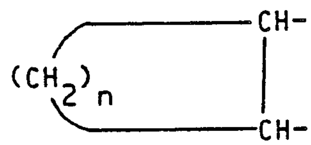 , 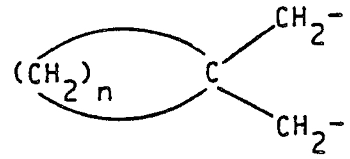 , 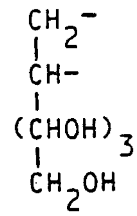

and 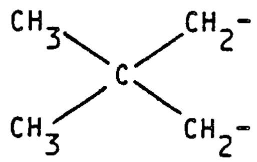

wherein n is the integer 3—5, inclusive; and A is selected from the group consisting of those of the formulae:

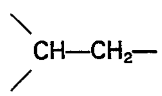 , 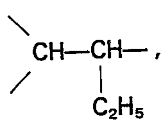 , 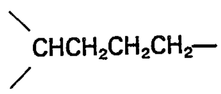 and 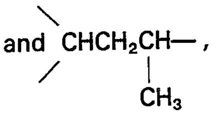 ,

which comprises reacting a diamine of the formula $2(R—NH)_2$ where R is as described above, with potassium tetrachloroplatinate in water giving a complex of the formula:

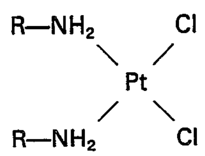

which is then reacted with silver nitrate giving an aqueous solution of a complex of the formula:

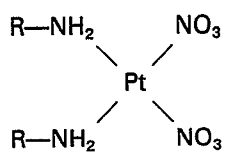

which is then reacted with a solution of a tricarboxylic acid of the formula:

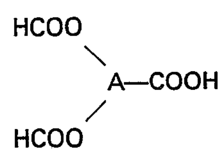

wherein A is as described above, in an aqueous base giving the above described products.

**Claims for the Contracting State: AT**

1. The use of a compound of the formula:

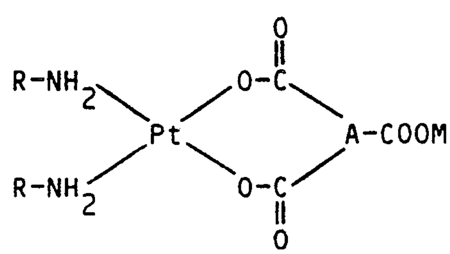

wherein M is hydrogen, sodium or potassium and R is hydrogen or alkyl($C_1$—$C_5$) or when taken together are selected from the group consisting of those of the formaulae:

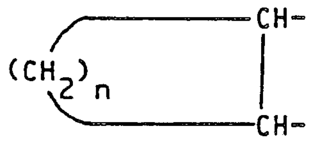 , 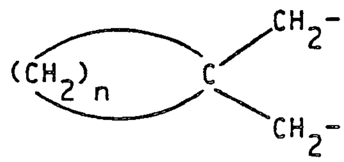 , 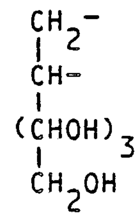

and 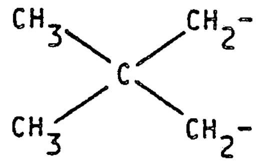

wherein n is the integer 3—5, inclusive; and A is selected from the group consisting of those of the formulae:

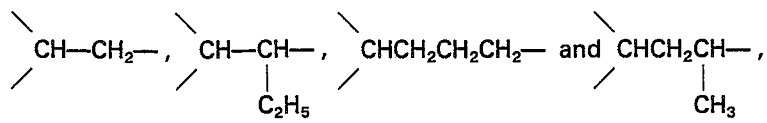

for the manufacture of a medicament for including the regression and/or palliation of leukemia and related cancers in mammals.

2. The use according to claim 1, wherein the compound is 2,2-dimethyl-1,3-propanediamine, compound with [1,1,2-ethanetricarboxylato(2-)-O[1],O[1]]platinum (1:1).

3. The use according to claim 1, wherein the compound is *trans*-(1)-1,2-cyclohexanediamine, compound with [1,1,2-ethanetricarboxylato(2-)-O[1],O[1]]platinum (1:1).

4. The use according to claim 1, wherein the compound is 1,1-cyclobutanedimethanamine, compound with [1,1,2-ethanetricarboxylato(2-)-O[1],O[1]]platinum (1:1).

5. The use according to claim 1, wherein the compound is *cis*(and *trans*)-1,2-cyclohexanediamine, compound with [1,1,2-ethanetricarboxylato(2-)-O[1],O[1]]platinum (1:1).

6. A process of producing a compound of the formula:

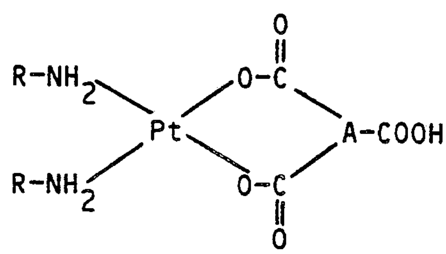

wherein R is hydrogen or alkyl($C_1$—$C_5$) or when taken together are selected from the group consisting of those of the formalae:

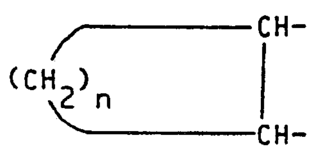 , 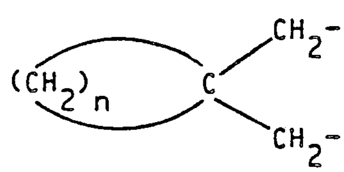 , 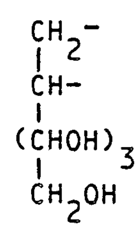

and 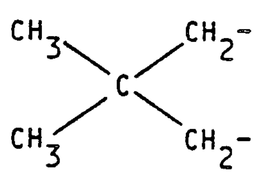

wherein n is the integer 3—5, inclusive; and A is selected from the group consisting of those of the formulae:

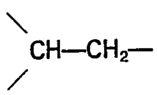 , 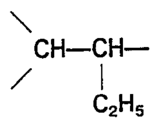 , 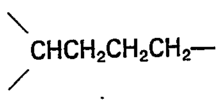 and 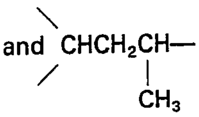 ,

which comprises reacting a diamine of the formula $2(R—NH)_2$ where R is as described above, with potassium tetrachloroplatinate in water giving a complex of the formula:

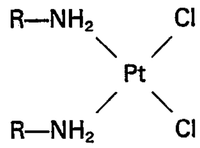 ,

which is then reacted with silver nitrate giving an aqueous solution of a complex of the formula:

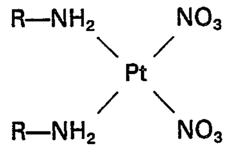 ,

which is then reacted with a solution of a tricarboxylic acid of the formula:

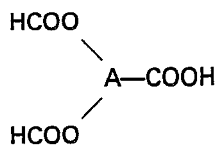 ,

wherein A is as described above, in an aqueous base giving the above described products.

**Patentansprüche für die Vertragsstaaten: BE CH DE FR GB IT LI NL SE**

1. Verbindung der Formel

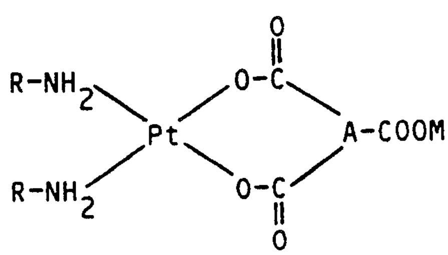

wobei M für Wasserstoff, Natrium oder Kalium steht und R Wasserstoff oder Alkyl($C_{1-5}$) ist oder, wenn sie zusammengefaßt sind, sie aus der Gruppe aus gewählt sind, bestehend aus Gruppen der Formeln:

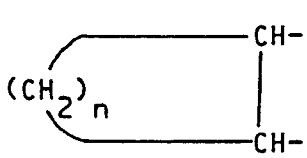 , 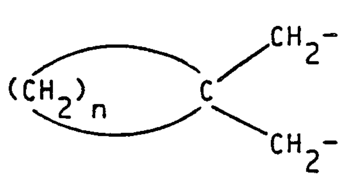 , 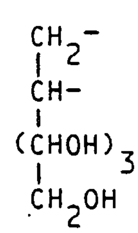

und 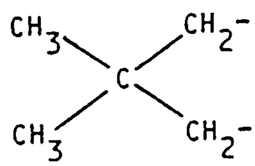

wobei n für eine ganze Zahl von 3—5 steht und A ausgewählt ist aus der Gruppe, bestehend aus Gruppen der Formeln:

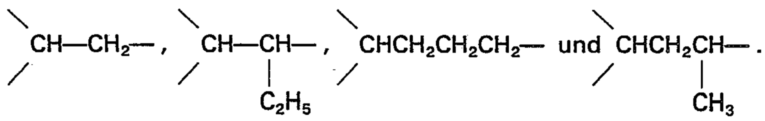

2. Verbindung gemäß Anspruch 1, nämlich 2,2-Dimethyl-1,3-propandiamin, verbindung mit [1,1,2-Ethantricarboxylato(2-)-O[1],O[1]]-Platin (1:1).

3. Verbindung gemäß Anspruch 1, nämlich *trans*-(1)-1,2-Cyclohexandiamin, verbunden mit [1,1,2-Ethantricarboxylato(2-)-O[1],O[1]]-Platin (1:1).

4. Verbindung gemäß Anspruch 1, nämlich 1,1-Cyclobutandimethanamin, verbunden mit [1,1,2-Ethantricarboxylato(2-)-O[1],O[1]]-Platin (1:1).

5. Verbindung gemäß Anspruch 1, nämlich cis(und trans)-1,2-Cyclohexandiamin, verbunden mit [1,1,2-Ethantricarboxylato(2-)-O[1],O[1]]Platin (1:1).

6. Verfahren zur Herstellung einer Verbindung der Formel:

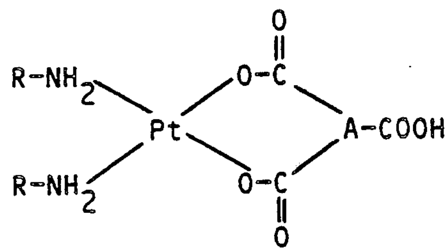

wobei R für Wasserstoff oder Alkyl($C_{1-5}$) steht oder, wenn sie zusammengefaßt sind, sie aus der Gruppe ausgewählt sind, bestehend aus Gruppen der Formeln:

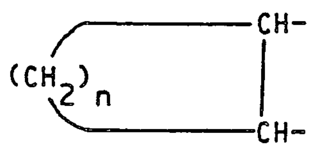 , 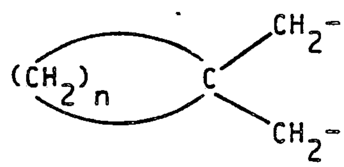 , 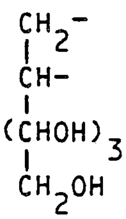

und 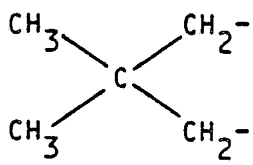

wobei n für eine ganze Zahl von 3—5 steht und A ausgewählt ist aus der Gruppe, bestehend aus Gruppen der Formeln:

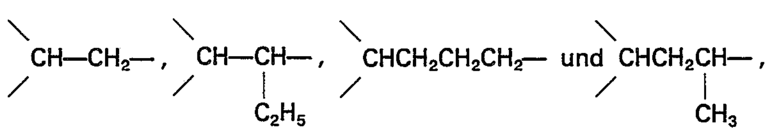

umfassend die Umsetzung eines Diamins der Formel 2(R—$NH_2$), wobei R wie oben beschrieben ist, mit Kaliumtetrachloroplatinat in Wasser, um einen Komplex der Formel:

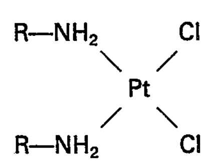

zu erhalten, der anschließend mit Silbernitrat umgesetzt wird, um eine wäßrige Lösung eines Komplexes der Formel:

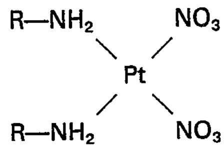

zu erhalten, der anschließend umgesetzt wird mit einer Lösung einer Tricarbonsäure der Formel:

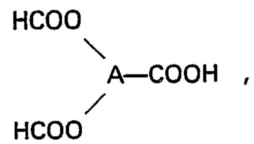

wobei A wie oben beschrieben ist, in einer wäßrigen Base, wobei man die oben beschriebenen Produkte erhält.

**Patentansprüche für den Vertragsstaat: AT**

1. Verwendung einer Verbindung der Formel

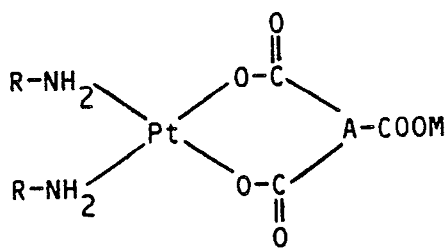

wobei M für Wasserstoff, Natrium oder Kalium steht und R Wasserstoff oder Alkyl($C_{1-5}$) ist oder, wenn sie zusammengefaßt sind, sie aus der Gruppe aus gewählt sind, bestehend aus Gruppen der Formeln:

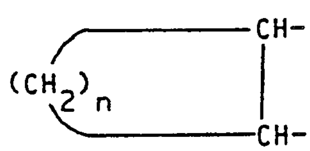 , 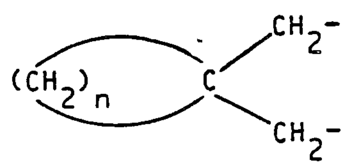 , 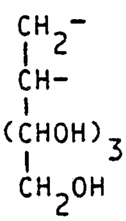

und 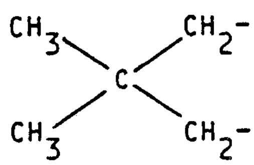

wobei n für eine ganze Zahl von 3—5 steht und A ausgewählt ist aus der Gruppe, bestehend aus Gruppen der Formeln:

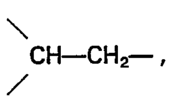, 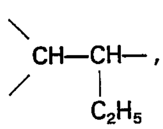, 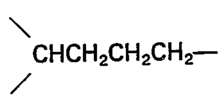 und 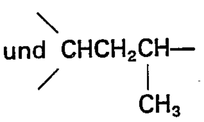

zur Herstellung eines Medikaments zur Induzierung des Rückgangs und/oder der Linderung von Leukämie und verwandten Krebserkrankungen bei Säugetieren.

2. Verwendung gemäß Anspruch 1, wobei die Verbindung 2,2-Dimethyl-1,3-propandiamin, verbindung mit [1,1,2-Ethantricarboxylato(2-)-O[1],O[1]]-Platin (1:1).

3. Verwendung gemäß Anspruch 1, wobei die Verbindung *trans*-(1)-1,2-Cyclohexandiamin, verbunden mit [1,1,2-Ethantricarboxylato(2-)-O[1],O[1]]-Platin (1:1), ist.

4. Verwendung gemäß Anspruch 1, wobei die Verbindung 1,1-Cyclobutandimethanamin, verbunden mit [1,1,2-Ethantricarboxylato(2-)-O[1],O[1]]-Platin (1:1), ist.

5. Verwendung gemäß Anspruch 1, wobei die Verbindung cis(und trans)-1,2-Cyclohexandiamin, verbunden mit [1,1,2-Ethantricarboxylato(2-)-O[1],O[1]]Platin (1:1) ist.

6. Verfahren zur Herstellung einer Verbindung der Formel:

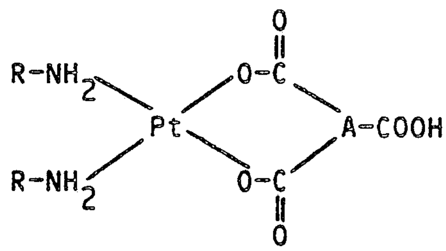

wobei R für Wasserstoff oder Alkyl($C_{1-5}$) steht oder, wenn sie zusammengefaßt sind, sie aus der Gruppe ausgewählt sind, bestehend aus Gruppen der Formeln:

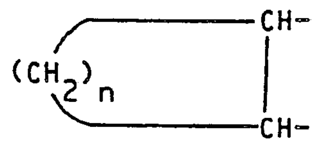 , 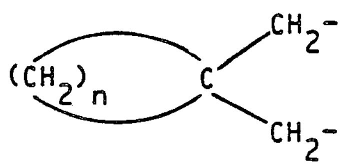 , 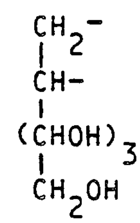

und 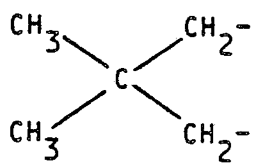

wobei n für eine ganze Zahl von 3—5 steht und A ausgewählt ist aus der Gruppe, bestehend aus Gruppen der Formeln:

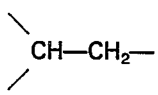 , 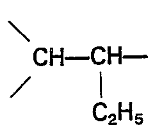 , 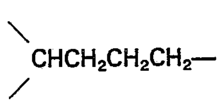 und 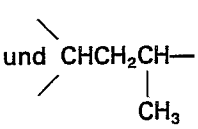 ,

umfassend die Umsetzung eines Diamins der Formel 2(R—$NH_2$), wobei R wie oben beschrieben ist, mit Kaliumtetrachloroplatinat in Wasser, um einen Komplex der Formel:

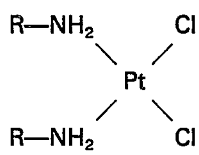

zu erhalten, der anschließend mit Silbernitrat umgesetzt wird, um eine wäßrige Lösung eines Komplexes der Formel:

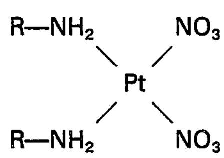

zu erhalten, der anschließend umgesetzt wird mit einer Lösung einer Tricarbonsäure der Formel:

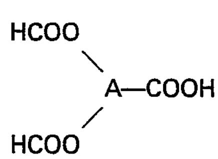 ,

wobei A wie oben beschrieben ist, in einer wäßrigen Base, wobei man die oben beschriebenen Produkte erhält.

**Revendications pour les Etats contractants: BE CH DE FR GB IT LI NL SE**

1. Un composé de formule:

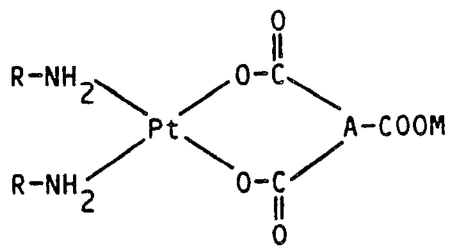

dans laquelle M est un atome d'hydrogène, de sodium ou de potassium et R est un atome d'hydrogène ou un groupe alkyle ($C_1$—$C_5$) ou les R pris ensemble sont choisis dans le groupe comprenant les formules suivantes:

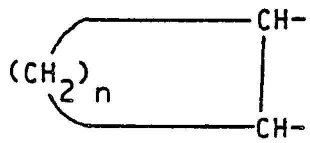 , 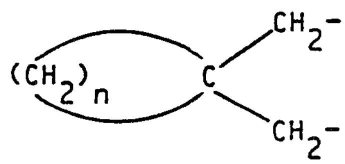 , 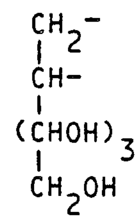

et 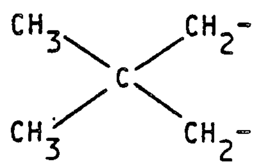

dans lesquelles n est un nombre entier de 3—5, inclus; et A est choisi dans le groupe comprenant les formules suivantes:

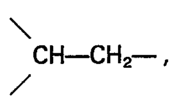 , 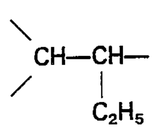 , 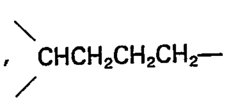 et 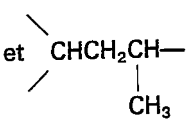 .

2. Le composé selon la revendication 1, qui est la 2,2-diméthyl-1,3-propanediamine, composé avec le [1,1,2-éthanetricarboxylato(2-)-O[1],O[1]]platine (1:1).

3. Le composé selon la revendication 1, qui est la *trans*(1)-1,2-cyclohexanediamine, composé avec le [1,1,2-éthanetricarboxylato(2-)-O[1],O[1]]platine (1:1).

4. Le composé selon la revendication 1, qui est la 1,1-éthanetricarboxylato(2-)-O[1],O[1]]platine (1:1).

5. Le composé selon la revendication 1, qui est la *cis*(et *trans*)-1,2-cyclohexanediamine, composé avec le [1,1,2-éthanetricarboxylato(2-)-O[1],O[1]]platine (1:1).

6. Un procédé de production d'un composé de formule:

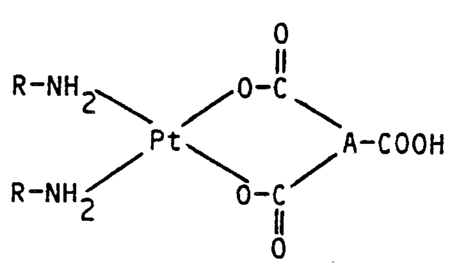

dans laquelle R est un atome d'hydrogène ou un groupe alkyle ($C_1$—$C_5$) ou les R pris ensemble sont choisis dans le groupe comprenant les formules:

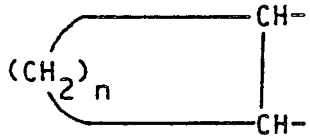, 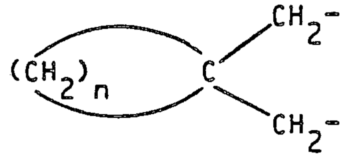, 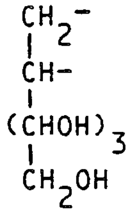

et 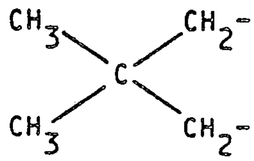

dans lesquelles n est un nombre entier de 3—5, inclus; et A est choisi dans le groupe comprenant les formules:

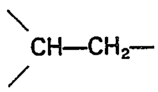, 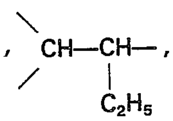, 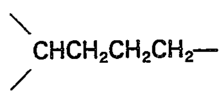 et 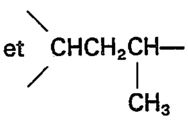,

qui comprend la réaction d'une diamine de formule $2(R—NH_2)$ dans laquelle R est comme décrit ci-dessus, avec le tétrachloroplatinate de potassium dans l'eau pour donner un complex de formule:

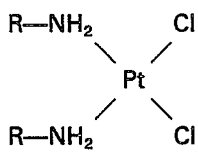

qui est mis à réagir avec du nitrate d'argent pour donner une solution aqueuse d'un complex de formule:

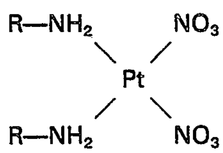

qui est ensuite mis à réagir avec une solution d'un acide tricarboxylique de formule:

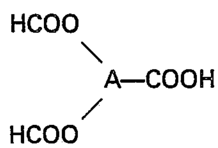 ,

dans laquelle A est comme décrit ci-dessus, dans une base aqueuse pour donner les produits décrits ci-dessus.

**Revendications pour l'Etat contactant: AT**

1. L'utilisation d'un composé de formule:

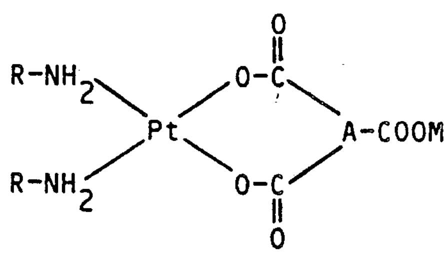

dans laquelle M est un atome d'hydrogène, de sodium ou de potassium et R est un atome d'hydrogène ou un groupe alkyle ($C_1$—$C_5$) ou les R pris ensemble sont choisis dans le groupe comprenant les formules:

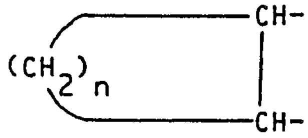, 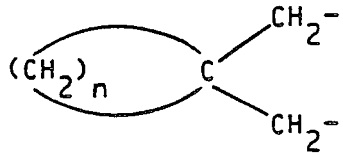, 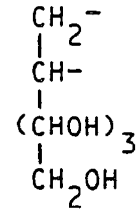

et 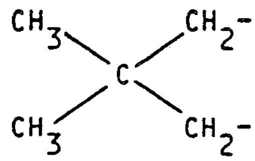

dans lesquelles n est un nombre entier de 3—5, inclus; et A est choisi dans le groupe comprenant les formules:

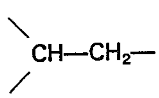, 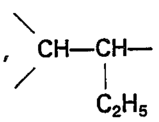, 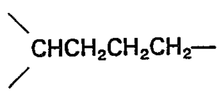 et 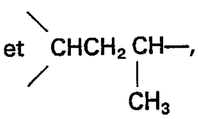,

pour la fabrication d'un médicament pour induire la régression et/ou l'atténuation de la leucémie et des cancers.

2. L'utilisation selon la revendication 1, selon laquelle le composé est la 2,2-diméthyl-1,3-propanediamine, composé avec le [1,1,2-éthanetricarboxylato(2-)-O[1],O[1]]platine (1:1).

3. L'utilisation selon la revendication 1, selon laquelle le composé est la *trans*(1)-1,2-cyclohexanediamine, composé avec le [1,1,2-éthanetricarboxylato(2-)-O[1],O[1]]platine (1:1).

4. L'utilisation selon la revendication 1, selon laquelle le composé est la 1,1-cyclobutane-diméthanamide, composé avec le [1,1,2-éthanetricarboxylato(2-)-O[1],O[1]]platine (1:1).

5. L'utilisation selon la revendication 1, selon laquelle le composé est la *cis*(et *trans*)-1,2-cyclohexanediamine, composé avec le [1,1,2-éthanetricarboxylato(2-)-O[1],O[1]]platine (1:1).

6. Un procédé de production d'un composé de formule:

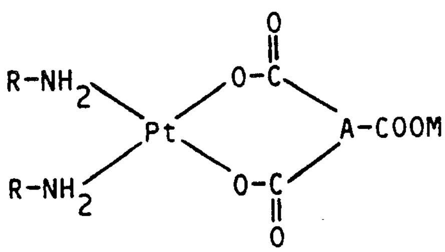

dans laquelle R est un atome d'hydrogène ou un groupe alkyle ($C_1$—$C_5$) ou bien les R pris ensemble sont choisis dans le groupe comprenant les formules:

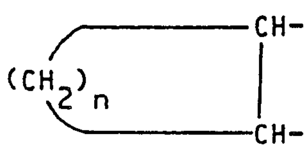, 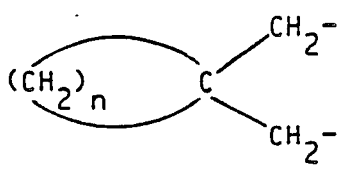, 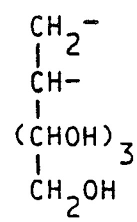

et 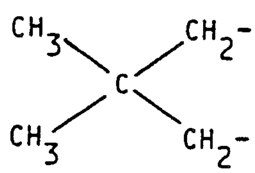

dans lesquelles n est un nombre entier de 3—5, inclus; et A est choisi dans le groupe comprenant les formules:

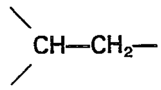, 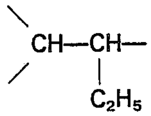, 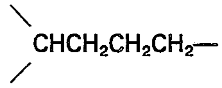 et 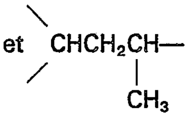,

qui comprend la réaction d'une diamine de formule $2(R—NH_2)$ dans laquelle R est comme décrit ci-dessus, avec le tétrachloroplatinate de potassium dans l'eau pour donner un complex de formule:

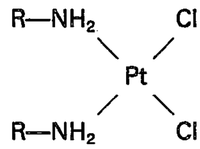

qui est ensuite mis à réagir avec le nitrate d'argent pour donner une solution aqueuse d'un complex de formule:

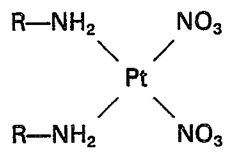

qui est ensuite mis à réagir avec une solution d'un acide tricarboxylique de formule:

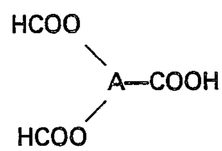 ,

dans laquelle A est comme décrit ci-dessus, dans une base aqueuse pour donner les produits décrits ci-dessus.